# EUROPÄISCHE PATENTSCHRIFT

(11) **EP 4 242 637 B1**
(45) Veröffentlichungstag und Bekanntmachung des Hinweises auf die Patenterteilung: **18.02.2026**
(21) Anmeldenummer: 23182756.9
(22) Anmeldetag: 02.03.2016
(51) Int. Cl.: G01N 21/21, G01P 13/00, G01N 21/47, G01N 33/50, G01N 21/49

(54) **VERFAHREN ZUR OPTISCHEN DETEKTION EINER BEWEGUNG IN EINER BIOLOGISCHEN PROBE MIT RÄUMLICHER AUSDEHNUNG**
METHOD FOR OPTICAL DETECTION OF A MOVEMENT IN A BIOLOGICAL SAMPLE WITH A SPATIAL EXTENT
PROCÉDÉ DE DÉTECTION OPTIQUE D'UN MOUVEMENT DANS UN ÉCHANTILLON BIOLOGIQUE À EXPANSION SPATIALE

(30) Priorität: 06.03.2015 DE 102015003019
(43) Veröffentlichungstag der Anmeldung: 13.09.2023
(62) Teilanmeldung aus: 16708082.9
(73) Patentinhaber: Universität des Saarlandes, 66123 Saarbrücken (DE); Fraunhofer-Gesellschaft zur Förderung der angewandten Forschung e.V., 80686 München (DE)
(72) Erfinder: Zimmermann, Heiko, 66280 Sulzbach (DE); Stracke, Frank, 66280 Sulzbach (DE); Le Harzic, Ronan, 66280 Sulzbach (DE)
(74) Vertreter: v. Bezold & Partner Patentanwälte - PartG mbB

(56) Entgegenhaltungen:
- EP-A1- 2 955 505
- WO-A1-2014/123156
- US-A- 5 061 075
- US-A1- 2006 105 357
- POMARICO J ET AL: "Compact device for assessment of microorganism motility", REVIEW OF SCIENTIFIC INSTRUMENTS, AIP, MELVILLE, NY, US, vol. 75, no. 11, 1 November 2004 (2004-11-01), pages 4727 - 4731, XP012071874, ISSN: 0034-6748, DOI: 10.1063/1.1809266
- J A COLE ET AL: "Laser speckle spectroscopy-a new method for using small swimming organisms as biomonitors", BIOIMAGING, vol. 4, no. 4, 1 December 1996 (1996-12-01), GB, pages 243 - 253, XP055277248, ISSN: 0966-9051, DOI: 10.1002/1361-6374(199612)4:4<243::AID-BIO3>3.3.CO;2-5
- P. P. DE TOMBE ET AL: "Force and velocity of sarcomere shortening in trabeculae from rat heart. Effects of temperature", CIRCULATION RESEARCH., vol. 66, no. 5, 1 May 1990 (1990-05-01), US, pages 1239 - 1254, XP055277139, ISSN: 0009-7330, DOI: 10.1161/01.RES.66.5.1239
- S M BAYLOR ET AL: "A LARGE BIREFRINGENCE SIGNAL PRECEDING CONTRACTION IN SINGLE TWITCH FIBRES OF THE FROG", J. PHYSIOL, 1 January 1977 (1977-01-01), pages 141 - 162, XP055278022, Retrieved from the Internet <URL:http://www.ncbi.nlm.nih.gov/pmc/articles/PMC1307751/pdf/jphysiol00824-0153.pdf>
- NODA NAOKI ET AL: "A new microscope optics for laser dark-field illumination applied to high precision two dimensional measurement of specimen displacement", REVIEW OF SCIENTIFIC INSTRUMENTS, AIP, MELVILLE, NY, US, vol. 79, no. 2, 14 February 2008 (2008-02-14), pages 23704 - 23704, XP012115026, ISSN: 0034-6748, DOI: 10.1063/1.2839914

## Beschreibung

Die Erfindung betrifft eine Vorrichtung zur optischen In-Vitro-Detektion einer Bewegung in einer biologischen Probe mit räumlicher Ausdehnung.

Aus der Praxis ist bekannt, dass ein Bedarf an einer Überwachung der aktiven Dynamik von geschlossenen und dreidimensionalen Zell- und Gewebekulturen in Bereichen wie der Entwicklungsbiologie, der Toxizitätstests und der pharmazeutischen Forschung besteht.

Beispielsweise werden aus embryonalen Stammzellen gezüchtete Gewebestücke, die sich zu einem Muskelgewebe differenziert haben, im Rahmen von Toxizitätstests genutzt, um die Schädlichkeit von einer zu prüfenden Substanz zu prüfen. Hierbei wird untersucht, ob eine auf das Muskelgewebe aufgebrachte Substanz die Muskelkontraktionen des Muskelgewebes beeinflusst, was ein Indikator für die Toxizität der Substanz sein kann. Hierfür sind Messverfahren erforderlich, um eine Bewegung, z. B. eine Kontraktion, in einer solchen dreidimensionalen biologischen Probe in Form eines Zellhaufens zu detektieren. Typische Durchmesser solcher Zellhaufen betragen 100 bis 400 µm, wobei auch Durchmesser im Millimeterbereich möglich sind.

Diese Studien werden derzeit in erster Linie durch visuelle Beobachtungen und selten durch Videomikroskopie mit anschließender Bildanalyse durchgeführt. Erstere sind zeitaufwändig und immer mit subjektiver Einschätzung verbunden. Letztere hat den Nachteil, dass komplexe abbildende Optiken und eine komplexe Bildanalyse verbunden mit einem erheblichen Rechenaufwand erforderlich sind. Nachteilig ist ferner ihre inhärente Empfindlichkeit gegenüber geringfügigen Verschiebungen.

Nichtoptische Methoden wie Impedanzmessungen funktionieren nur im Kontakt zur Probe. Wenn die Probeform jedoch von der Ebene (adhärente Monolage) abweicht oder gar dreidimensional ist, noch dazu frei in einem Medium schwimmt, sind die vorgenannten Techniken nicht anwendbar.

Automatisierte bildgebende Verfahren haben den Nachteil, dass z. B. bei einer Schärfentiefe von 10 µm und der vorstehend genannten typischen Größe der Zellhaufen von 100 bis 400 µm 10 bis 40 Bildebenen der Probe durchgemessen werden müssten. Bei einer typischen Mindestmessdauer von ca. 10 Sekunden, um eine Bewegung detektieren zu können, und der zusätzlich für die Neupositionierung bzw. Fokussierung benötigten Zeit sind derartige Verfahren nicht geeignet, schnell eine Vielzahl von Proben zu überwachen.

Eine serielle Messung von großen Probenzahlen ist auf Grund der durch die Zeitskalen der biologischen Dynamik recht langen Beobachtungsdauer generell nicht angezeigt. In der Praxis besteht jedoch der Bedarf, eine derartige Bewegungsdetektion an einer Vielzahl voneinander getrennter, z. B. in einer Multiwell-Platte, auch als Mikrotiterplatte bezeichnet, z. B. mit in 96 oder 384 Kavitäten (engl. Wells) gelagerten Proben, durchzuführen. Bildgebende Verfahren sind für eine parallele Messung einer Vielzahl solcher Proben nicht geeignet, da es aus geometrischen und bauraumtechnischen Gründen schwierig zu realisieren ist, an jeder Kavität der Multiwell-Platte eine bildgebende optische Vorrichtung anzuordnen.

Der Artikel: J A COLE ET AL: "Laser speckle spectroscopy-a new method for using small swimming organisms as biomonitors", BIOIMAGING, Bd. 4, Nr. 4, Dezember 1996, Seiten 243-253 offenbart eine punktuelle Messung eines Speckle-Musters mit anschließender Fourier-Transformation (FFT) zur Analyse der Bewegung von Mikro-Organismen.

Es ist somit eine Aufgabe der Erfindung, eine Vorrichtung zur Detektion einer Bewegung in einer biologischen Probe mit räumlicher Ausdehnung bereitzustellen, mit der Nachteile herkömmlicher Vorrichtungen vermieden werden können.

Diese Aufgabe wird durch die Vorrichtung mit den Merkmalen des unabhängigen Anspruchs gelöst. Vorteilhafte Ausführungsformen und Anwendungen der Erfindung ergeben sich aus den abhängigen Ansprüchen und werden in der folgenden Beschreibung unter teilweiser Bezugnahme auf die Figuren näher erläutert.

Die Erfindung beruht auf der technischen Erkenntnis, dass für eine kontaktlose Überwachung optische Methoden am geeignetsten sind und dass, da der genaue Ort einer möglichen Bewegung in der Probe nicht von vornherein bekannt ist, es erforderlich ist, die gesamte Probe zu beleuchten. Da die Probe in der Regel eine erhebliche Ausdehnung in der Tiefe, d. h. größer als die Schärfentiefe einer Abbildungsoptik, besitzt, müssen die Wechselwirkungen des Lichts mit der Probe auf seiner gesamten Strecke durch die Probe kumuliert gemessen werden. Transmissionsmethoden sind daher ungeeignet, da hier gerade der nicht-wechselwirkende Teil des Lichts gemessen würde und die zu erwartenden Schwankungen von einem hohen Hintergrund und Rauschen begleitet wären, der die Technik unempfindlich machen würde. Der erfindungsgemäße Ansatz beruht daher auf der Erfassung der Streu-, Polarisations- und/oder Beugungsstrahlung der belichteten Probe, wobei nach den durch die Probenbewegung hervorgerufenen Schwankungen in dem Teil des Lichts gesucht wird, der durch Wechselwirkung mit der Probe in ihrer Strahlrichtung, ihrem Polarisationszustand und/oder ihrem Beugungsmuster verändert wurde. Dazu ist es vorteilhaft, das transmittierende Licht durch geeignete Filter von der Streu-, Polarisations- und/oder Beugungsstrahlung abzutrennen. Im Rahmen der vorliegenden Erfindung wird statt des Begriffs "Strahlung" auch der Begriff "Licht" verwendet. Beide Begriffe sind im Rahmen der vorliegenden Erfindung als gleichbedeutend anzusehen und umfassen elektromagnetische Strahlung im sichtbaren, IR- und UV-Bereich.

Außerhalb der Erfindung wird ein Verfahren zur optischen In-Vitro-Detektion einer Bewegung in einer biologischen Probe mit räumlicher Ausdehnung bereitgestellt.

Das genannte Verfahren umfasst das Bereitstellen einer Aufnahme für die Probe, einer Lichtstrahlenquelle, einer Optik und eines Detektors. Hierbei ist die Optik ausgebildet, die ganze Probe in der Aufnahme mit von der Lichtstrahlenquelle ausgehender Strahlung zu beleuchten und zumindest einen Teil der Strahlung der Lichtstrahlenquelle, die an einer beliebigen Stelle innerhalb der Probe durch eine Wechselwirkung mit der Probe in ihrer Strahlrichtung, ihrem Polarisationszustand und/oder ihrem Beugungsmuster verändert wird, auf eine Detektionsfläche des Detektors zu leiten, so dass die Wechselwirkungen der von der Lichtstrahlenquelle ausgesandten Strahlung mit der Probe auf seiner gesamten Strecke durch die Probe kumuliert gemessen werden. Der Detektor ist ausgebildet, in Abhängigkeit von der detektierten Strahlung ein Messsignal zu erzeugen, dessen zeitlicher Verlauf einen zeitlichen Verlauf der Intensität der detektierten Strahlung angibt und/oder aus dem der zeitliche Verlauf der Intensität der detektierten Strahlung ableitbar ist.

Das genannte Verfahren umfasst ferner das Beleuchten der Probe mit Strahlung der Lichtstrahlenquelle und das Detektieren einer Bewegung in der biologischen Probe in Abhängigkeit von einer zeitlichen Veränderung des Messsignals.

Ein besonderer Vorzug dieses Ansatzes ist, dass aus der Messgröße des Verfahrens direkt die Probendynamik ableitbar ist, da die von einer Probenbewegung hervorgerufenen Schwankungen in dem Teil des Lichts, der durch Wechselwirkung mit der Probe in Strahlrichtung, Polarisationszustand und/oder Beugungsmuster verändert wurde, direkt als Schwankungen in dem Messsignal sichtbar sind. Somit kann auf eine aufwändige Verarbeitung der Messdaten, wie dies bei bildgebenden Verfahren der Fall ist, verzichtet werden.

Beispielsweise kann das Verfahren ausgeführt sein, eine Bewegung in der Probe zu detektieren, falls eine Veränderung des Messsignals einen vorbestimmten Schwellenwert übersteigt. Bei der Überprüfung von Kontraktionen in einem Muskelgewebe kann eine Bewegung ferner detektiert werden, falls die zeitliche Veränderung des Messsignals eine Periodizität aufweist.

Das Verfahren kann unter Verwendung vergleichsweise einfacher optischer Elemente durchgeführt werden. Eine Optik zur Fokussierung auf einzelne Bildebenen und zum sukzessiven Abtasten des Probenvolumens ist nicht notwendig. Daher kann die Vorrichtung zur Durchführung des Verfahrens kostengünstig und baulich kompakt ausgeführt sein.

Aufgrund der einfachen Auswertung des Messsignals und des baulich kompakten Aufbaus ist das Verfahren auch für die parallele Überwachung einer Vielzahl von Proben geeignet und lässt sich prozesseffizient in Screening-Umgebungen bzw. in automatisierte Hochdurchsatzverfahren, z. B. High-Throughput Screening-Verfahren, integrieren.

Gemäß einer besonders bevorzugten Ausführungsvariante ist der Detektor einkanalig ausgeführt bzw. gibt der Detektor ein einkanaliges Messsignal aus. Das Messsignal gibt vorzugsweise nur die pro Zeiteinheit auf die Detektorfläche eintreffende Strahlungsintensität an. Eine Bewegung in der Probe kann somit direkt anhand einer zeitlichen Veränderung bzw. Schwankung des Signals detektiert werden.

Der Detektor ist vorzugsweise ein nicht bildgebender Detektor oder ein Detektor mit nicht ortsaufgelöstem Messsignal, z. B. ein nicht ortsauflösender Photodetektor, beispielsweise eine Photodiode. Derartige Detektoren sind kompakt und kostengünstig.

Unter einer biologischen Probe mit räumlicher Ausdehnung wird eine dreidimensionale biologische Probe, z. B. in Form einer dreidimensionalen Zell- und/oder Gewebekultur bzw. eines Zellhaufens, verstanden.

Der Durchmesser der biologischen Probe kann mindestens 50 Mikrometer (µm) in mindestens einer Raumrichtung, weiter vorzugsweise mindestens 50 Mikrometer in allen Raumrichtungen betragen und beträgt oft mehr als 100 µm in allen Raumrichtungen.

Der Durchmesser der Probe liegt vorzugsweise im Bereich von 100 µm bis 5 mm, weiter vorzugsweise im Bereich von 100 µm bis 1 mm. Die biologische Probe kann insbesondere eine Probe aus Muskelgewebe und/oder aus lebenden Zellen sein, d. h. aus Zellen, die eine Aktivdynamik aufweisen, d. h. Zellen, die eine Bewegung auslösen können.

Unter der Detektion einer Bewegung in der biologischen Probe soll insbesondere eine Bewegung innerhalb der Probe oder eine Bewegung eines Probenbestandteils der biologischen Probe verstanden werden. Mit anderen Worten sollen allgemein dynamische Phänomene in bzw. innerhalb biologischer Proben mit räumlicher Ausdehnung detektiert werden können. Bei Zellkulturen aus Muskelzellen können derartige Bewegungen beispielsweise durch Kontraktion einzelner Muskelzellen ausgelöst werden.

Bei der biologischen Probe kann es sich jedoch auch um eine Probe aus freischwimmenden Mikroorganismen, beispielsweise Spermien, handeln. In diesem Fall kann das Verfahren zur Detektion der Bewegung der freischwimmenden Mikroorganismen genutzt werden, beispielsweise im Falle von Spermien zur Bestimmung der Spermienmotilität.

Die Optik kann eine auf der Beleuchtungsseite angeordnete Beleuchtungsoptik umfassen, mittels der die Strahlung der Lichtstrahlenquelle auf die ganze Probe geleitet wird, um die Probe vollständig und möglichst gleichmäßig zu beleuchten.

Die Optik kann ferner eine Detektionsoptik umfassen, mittels der das von der Probe ausgesandte Licht, das durch eine Wechselwirkung mit der Probe in ihrer Strahlrichtung, ihrem Polarisationszustand und/oder ihrem Beugungsmuster verändert wird, auf eine Detektionsfläche des Detektors geleitet wird.

Diese funktionale Eigenschaft der Optik kann dabei unter Verwendung eines oder mehrerer zweckmäßig angeordneter und ausgestalteter bekannter optischer Bauelemente und Komponenten, wie z. B. Filter, Linsen, Blenden, refraktiver Elemente etc., realisiert werden, was nachfolgend anhand weiterer Ausführungsbeispiele erläutert wird.

Eine vorteilhafte Ausführungsvariante sieht hierbei vor, dass die Optik ausgebildet ist und/oder der Detektor relativ zum Beleuchtungsstrahlengang und der Probe so angeordnet ist, dass keine Strahlengänge existieren, bei denen durch die Probe transmittierte Strahlung der Lichtstrahlenquelle auf den Detektor trifft und/oder bei denen Licht der Lichtstrahlenquelle unter Umgehung der Probe auf den Detektor trifft.

Gemäß dieser Ausführungsvariante trifft somit nur dasjenige Licht der Lichtstrahlenquelle auf den Detektor, das durch eine Wechselwirkung mit der Probe in seiner Strahlrichtung, seinem Polarisationszustand und/oder Beugungsmuster verändert wurde, während die Optik verhindert, dass Transmissionsstrahlung oder die Probe umgehende Strahlung die Detektionsfläche des Detektors trifft. Dadurch werden störende Hintergrundsignale reduziert und die Empfindlichkeit der Messung erhöht.

Bei der Vorrichtung der Erfindung wird eine Bewegung in der Probe anhand einer Veränderung des Beugungsmusters erkannt. Gemäß der Erfindung erzeugt die Lichtstrahlenquelle kohärentes Licht. Ferner ist die Optik ausgebildet, z. B. mittels eines Raumfilters, einen Randbereich eines Beugungsmusters, das von durch die Probe gebeugtem Licht der Lichtstrahlenquelle erzeugt wird, auf den Detektor abzubilden. Eine Bewegung innerhalb der Probe erzeugt eine Änderung des Beugungsmusters, beispielsweise eines Speckle-Musters. Untersuchungen im Rahmen der Erfindung haben gezeigt, dass die Änderung des Beugungsmusters in seinem Zentrum schwierig zu messen ist, da die relative Änderung der Strahlungsintensität klein ist. Im Randbereich ist die Änderung jedoch zuverlässig zu erkennen und kann z. B. zu einer kurzzeitigen Änderung von einem lokalen Beugungsmaximum zu einem lokalen Beugungsminimum oder vice versa im Beugungsmuster führen. Bei einem Beugungsmuster enthält jeder Punkt des Musters die Beugungsinformation der gesamten Probe.

Bei einer vorteilhaften Variante dieser Ausgestaltungsform umfasst die Optik eine Lochblende, die so zwischen der Probe und dem Detektor angeordnet ist, dass ein Loch der Lochblende an dem Randbereich des von der Probe erzeugten Beugungsmusters angeordnet ist.

Unter einer Lochblende wird eine lochförmige Öffnung, vorzugsweise eine kleine lochförmige Öffnung verstanden und vorzugsweise ohne Linse. Lochblenden dienen dem örtlich begrenzten Aufsammeln von Licht. Zum gleichen Zweck werden insbesondere in konfokalen Mikroskopen seit langer Zeit die Stirnseiten von optischen Fasern genutzt.

Als Randbereich des Beugungsmusters sollen vorzugsweise alle Beobachtungswinkel gelten, in den kein transmittiertes Licht empfangen wird. Das Beugungsmuster wird durch positive und negative Interferenz von Lichtwellen, die an Objekten, hier der Probe, gebeugt wurden, hervorgerufen. Ob positiv oder negativ interferiert wird, hängt von Objektgröße, Wellenlänge des Lichts und Beobachtungswinkel ab. Licht, das die Probe ohne Wechselwirkung durchläuft, d. h. transmittiertes Licht, ballistische Photonen, hat den Beobachtungswinkel 0° und trifft in der Mitte des Beugungsmusters auf. Das Beugungsmuster wird durch das transmittierte Licht überstrahlt und das S/B (S/B: signal-to-background, Signal-Hintergrund)-Verhältnis und das S/N (S/N: signal-to-noise, Signal-Rausch)-Verhältnis fallen drastisch. Als Randbereich des Beugungsmusters sollen daher vorzugsweise alle Beobachtungswinkel gelten, in den kein transmittiertes Licht empfangen wird. Da hier die ganze Probe bestrahlt wird und die Beleuchtung nicht kollimiert erfolgt, ist der "Empfangsbereich" der transmittierten Strahlung größer als nur ein Punkt.

Gemäß einer weiteren Variante dieser Ausgestaltungsform kann die Lochblende auch mehrere Löcher aufweisen, die relativ zum Beugungsmuster so angeordnet sind, dass sie sich in einem Randbereich des Beugungsmusters befinden. Hierbei sind die Löcher so anzuordnen, dass die Überlagerung der Beugungsstrahlung, die durch die Löcher auf den Detektor trifft, den Beugungseffekt verstärkt und nicht verschlechtert.

Gemäß einer weiteren Variante der Ausgestaltungsform, die eine Bewegung in der Probe anhand einer Veränderung des Beugungsmusters erkennt, kann die Optik eine zwischen der Lichtstrahlenquelle und der Probe angeordnete Blende umfassen, die so ausgeführt ist, dass eine durch eine Blendenöffnung der Blende austretende Strahlung der Lichtstrahlenquelle nicht direkt, d. h. unter Umgehung der Probe, auf das Loch der Lochblende trifft. Ferner kann die Optik ein zwischen der Lichtstrahlenquelle und der Probe angeordnetes refraktives optisches Element, d. h. ein die Strahlung brechendes Element, z. B. eine konvexe Linse oder ein Prisma, aufweisen, das so ausgeführt ist, dass durch das refraktive Element abgelenkte Strahlung nicht direkt, d. h. unter Umgehung der Probe, auf das Loch der Lochblende trifft. Diese Varianten stellen ein kostengünstiges und einfach zu justierendes Beispiel für eine Optik dar, die nur die gebeugte Strahlung zum Loch der Lochblende leitet.

Gemäß einer weiteren nicht erfindungsgemäßen Ausgestaltungsform wird eine Bewegung in der Probe anhand einer durch die Bewegung verursachten Schwankung von polarisiertem Licht erkannt. Gemäß dieser Ausgestaltungsform umfasst die Optik einen ersten Polarisationsfilter und einen zweiten Polarisationsfilter, die unterschiedliche Polarisationsrichtungen aufweisen, wobei der erste Polarisationsfilter zwischen Lichtstrahlenquelle und Probe und der zweite Polarisationsfilter zwischen Probe und Detektor angeordnet ist. Eine Bewegung in der Probe führt zu einer geänderten Wechselwirkung des polarisierten Lichts mit der Probe und zu einer Änderung der Polarisationszustände, was zu einer Schwankung im Detektorsignal führt.

Hierbei können der Detektor und der zweite Polarisationsfilter in Bezug auf die Lichtstrahlenquelle auf der gegenüberliegenden Seite der Probe, seitlich von der Probe oder auf der gleichen Seite wie die Lichtstrahlenquelle angeordnet sein.

Gemäß einer weiteren nicht erfindungsgemäßen Ausgestaltungsform wird eine Bewegung in der Probe anhand einer durch die Bewegung verursachten Schwankung des an der Probe gestreuten Lichts erkannt. Hierbei kann der der Detektor zur Epidetektion von Streulicht der Probe auf der gleichen Seite der Probe (1) wie die Lichtstrahlenquelle angeordnet sein. Alternativ kann der der Detektor zur Detektion seitlicher Streustrahlung der Probe in Bezug auf die Richtung des Beleuchtungsstrahlengangs schräg und/oder seitlich von der Probe angeordnet sein.

Diese Varianten bieten den Vorteil, dass keine optischen Komponenten wie z. B. Blenden notwendig sind, die verhindern, dass Transmissionsstrahlung auf den Detektor trifft.

Ferner kann der Detektor zur Durchlichtdetektion von Streulicht der Probe in Durchlichtrichtung zur Probe angeordnet sein. Gemäß dieser Variante umfasst die Optik eine zwischen der Lichtstrahlenquelle und der Probe angeordnete Blende, die ausgebildet ist, im Beleuchtungsstrahlengang Lichtstrahlen, die als durch die Probe transmittierte Strahlen auf den Detektor treffen würden, zu blockieren. Alternativ oder zusätzlich kann die Optik ein zwischen der Lichtstrahlenquelle und der Probe angeordnetes refraktives optisches Element umfassen, das ausgebildet ist, eine Richtung des Beleuchtungsstrahlengangs so zu verändern, dass durch die Probe transmittierte Lichtstrahlen nicht auf den Detektor treffen.

Ferner ist es vorteilhaft, wenn die Optik einen vor dem Detektor angeordneten Bandpassfilter aufweist, der zur Raumlichtunterdrückung ausgebildet ist. Dadurch kann die Empfindlichkeit der Detektion weiter erhöht werden.

Gemäß einer weiteren vorteilhaften Variante umfasst die Optik, insbesondere die Beleuchtungsoptik, ein Axicon. Die Verwendung eines Axicons bietet den Vorzug einer homogeneren Ausleuchtung räumlich "tiefer" Proben, verglichen mit konvexen Linsen, da sich der Fokus eines Axicons entlang der optischen Achse erstreckt - statt nur in einem Punkt. Ein weiterer Vorzug eines Axicons ist die einfache räumliche Filterung des eingestrahlten (nicht beeinflussten) Lichts, da dieses mit einem konstanten Winkel zur optischen Achse gebrochen wird.

Eine Möglichkeit der Realisierung sieht vor, dass sich die Probe auf einer Trägermatrix befinden kann. Die Aufnahme der Probe kann somit eine Trägermatrix, vorzugsweise ein Biopolymer, umfassen. Die Probe kann sich ferner in einem hängendem Tropfen befinden. Die Aufnahme der Probe kann somit einen hängenden Tropfen umfasst. Ferner kann die Aufnahme der Probe als eine sich in einem hängenden Tropfen befindliche Trägermatrix, vorzugsweise ein Biopolymer wie z. B. Alginat, ausgeführt sein.

Für die Anwendung der Vorrichtung der Erfindung in Screening-Umgebungen kann die Aufnahme für die Probe eine Kavität einer Multiwell-Platte (Mikrotiterplatte) sein. Weiterhin kann die Aufnahme eine Kavität einer Multiwell-Platte sein, die zur Ausbildung eines hängenden Tropfens an den einzelnen Kavitäten ausgebildet ist (sog. Hängender-Tropfen-Multiwell-Platte). Solche Hängender-Tropfen-Multiwell-Platten werden beispielsweise von der Firma Insphero AG, CH-8952 Schlieren unter der Bezeichnung "GravityPLUS^{™} 3D Culture and Assay Platform" angeboten. Auch die Patentschrift EP 2342317 B1 offenbart eine solche Platte.

Vorstehend wurde bereits erwähnt, dass sich die Vorrichtung der Erfindung für die parallele Überwachung einer Vielzahl von Proben eignet, z. B. von Proben, die im Rahmen von automatisierten Hochdurchsatzverfahren untersucht werden sollen.

Die Erfindung sieht daher vor, dass mit diesem eine parallele optische Detektion einer Bewegung in mehreren voneinander getrennten biologischen Proben durchgeführt wird. Hierbei ist die Aufnahme für die biologischen Proben vorzugsweise eine Multiwell-Platte, die eine Mehrzahl von in Reihen und Spalten angeordneten Kavitäten zur Aufnahme der Proben aufweist. Der Detektor ist als Detektorarray, vorzugsweise als Photodiodenarray, ausgeführt, wobei ein Rasterabstand der einzelnen Detektoren einem Rasterabstand der Kavitäten der Multiwell-Platte entspricht.

Die Lichtstrahlenquelle ist ausgeführt, die einzelnen Kavitäten zu beleuchten. Gemäß der Erfindung ist die Lichtstrahlenquelle zur Beleuchtung der Proben in den Kavitäten als ein Laserdiodenarray ausgeführt, wobei ein Rasterabstand der einzelnen Laserdioden dem Rasterabstand der Kavitäten der Multiwell-Platte entspricht. Ein Laserdiodenarray stellt eine platzsparende und energieeffiziente Beleuchtungsquelle dar.

Um einen guten Wärmeabtransport zu ermöglichen, kann die Halterung des Laserdiodenarrays aus einem wärmeleitfähigen Material, vorzugsweise aus Aluminium, ausgeführt sein. Weiterhin kann die Optik gemäß dieser Variante ein Linsenarray, z. B. ein Mikrolinsenarray, umfassen, wobei jede Linse des Linsenarrays einer der Laserdioden zugeordnet ist und die Linsen das Licht der Laserdioden in die Kavitäten leiten.

Anstatt eines Laserdiodenarrays als Lichtstrahlenquelle kann außerhalb der Erfindung die Lichtstrahlenquelle als herkömmliche Lichtquelle (Laser, Bogenlampe etc.) ausgeführt sein, wobei das Licht der Lichtstrahlenquelle zur Beleuchtung der Proben über ein Lichtfaserbündel in die einzelnen Kavitäten, die die Proben enthalten, eingekoppelt wird. Jede Lichtfaser ist dabei einer Kavität zugeordnet. Dies bietet den Vorteil, dass die Lichtquelle ausreichend beabstandet zur Probe betrieben werden kann, um und eine zu starke Wärmeentwicklung in Probennähe zu vermeiden.

Ferner besteht gemäß einer weiteren alternativen Variante die Möglichkeit, die Multiwell-Platte mit einer Lichtstrahlenquelle flächig zu beleuchten, was eine einfache Umsetzungsvariante darstellt, jedoch Energieeffizienznachteile hat, da die Lichtstrahlenquelle entsprechend leistungsfähig sein muss. Vorteilhaft ist es hierbei, wenn das Licht über eine zweckmässig ausgeführte Kondensoroptik und ggf. winkelabhängige Durchlassfilter parallelisiert und gezielt auf die Kavitäten geleitet wird.

Gemäß der Erfindung wird eine Vorrichtung zur kontaktfreien In-Vitro-Detektion einer Bewegung in einer biologischen Probe mit räumlicher Ausdehnung wie im Anspruch 1 bereitgestellt. Die Vorrichtung umfasst eine Aufnahme für die biologische Probe, eine Lichtstrahlenquelle, einen Detektor und eine Optik, die ausgebildet ist, die ganze Probe in der Aufnahme mit von der Lichtstrahlenquelle ausgehenden Strahlung zu beleuchten und zumindest einen Teil der Strahlung der Lichtstrahlenquelle, die an einer beliebigen Stelle innerhalb der Probe durch eine Wechselwirkung mit der Probe in ihrem Beugungsmuster verändert wurde, auf eine Detektionsfläche des Detektors zu leiten. Der Detektor ist ausgebildet, in Abhängigkeit von der detektierten Strahlung ein Messsignal zu erzeugen, das den zeitlichen Verlauf der Intensität der detektierten Strahlung angibt und/oder aus dem der zeitliche Verlauf der Intensität der detektierten Strahlung ableitbar ist.

Die Vorrichtung weist ferner eine Auswerteeinheit auf, die eingerichtet ist, eine Bewegung in der biologischen Probe durch Anzeige und/oder Auswertung einer zeitlichen Veränderung der detektierten Strahlung zu detektieren.

Zur Vermeidung von Wiederholungen sollen rein verfahrensgemäß offenbarte Merkmale auch als vorrichtungsgemäß offenbart gelten. Die vorgenannten Aspekte und erfindungsgemäßen Merkmale, insbesondere im Hinblick auf die Ausbildung der Optik, des Detektors, der Aufnahme, z. B. als Multiwell-Platte, hängender Tropfen oder Hängender-Tropfen-Multiwell-Platte, und die Lichtstrahlenquelle gelten somit auch für die Vorrichtung.

Die zuvor beschriebenen bevorzugten Ausführungsformen und Merkmale der Erfindung sind beliebig miteinander kombinierbar. Weitere Einzelheiten und Vorteile der Erfindung werden im Folgenden unter Bezug auf die beigefügten Zeichnungen beschrieben. Es zeigen:
- Figur 1: eine stark schematisierte Darstellung eines Verfahrens und einer Vorrichtung gemäß einer Ausführungsform zur Verständnis der Erfindung;
- Figuren 2A-2E: Ausführungsformen außerhalb der Erfindung, die Streulicht der Probe zur Bewegungsdetektion verwenden;
- Figuren 3A-3C: Ausführungsformen außerhalb der Erfindung, die polarisiertes Licht zur Bewegungsdetektion verwenden;
- Figuren 4A-4C: Ausführungsformen zur Verständnis der Erfindung, die ein Beugungsmuster der Probe zur Bewegungsdetektion verwenden;
- Figur 5A eine: Probe in Form eines Herzmuskelgewebemodells auf einer Trägermatrix;
- Figur 5B: Darstellung eines Speckle-Musters an zwei aufeinanderfolgenden Zeitpunkten; und
- Figur 5C: einen zeitlichen Verlauf eines beispielhaften Messsignals.

Gleiche Teile sind in den Figuren mit denselben Bezugszeichen versehen und werden nicht gesondert beschrieben.

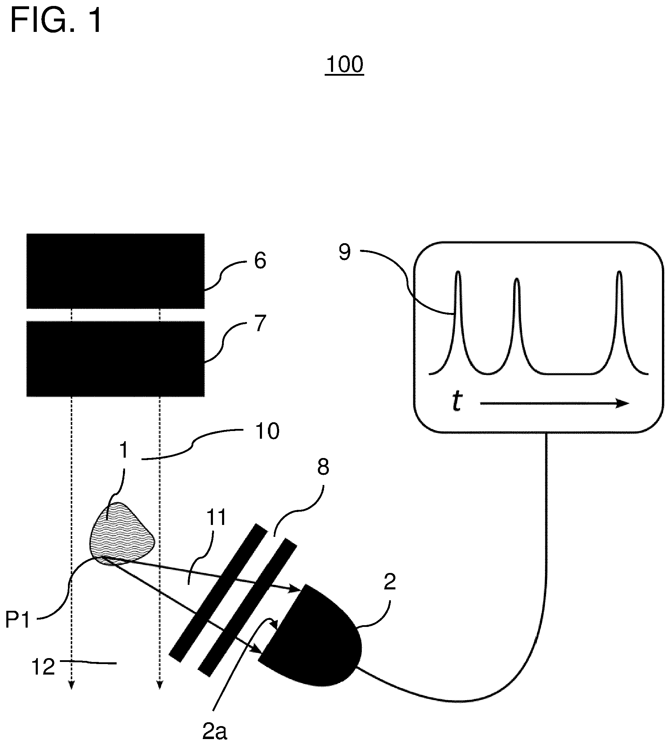

Figur 1 zeigt eine stark schematisierte Darstellung eines Verfahrens und einer Vorrichtung gemäß einer Ausführungsform zur Verständnis der Erfindung.

Zur Durchführung des Verfahrens wird eine Vorrichtung 100 zur optischen In-Vitro-Detektion einer Bewegung in einer biologischen Probe 1 mit räumlicher Ausdehnung bereitgestellt.

Die Vorrichtung 100 umfasst eine Aufnahme (nicht dargestellt) für die dreidimensionale Probe 1, eine Lichtstrahlenquelle 6, eine Optik 7, 8 und einen Detektor 2.

Die Aufnahme ist nicht auf eine bestimmte Art von Aufnahmen beschränkt, sondern kann je nach Anwendungszweck und Art der Probe zweckmäßig, z. B. als Träger, Trägerplatte, als Gefäß, als Kavität einer Multiwellplatte, oder als Trägermatrix in Form eines Biopolymers, z. B. Alginat, auf dem die Probe gezüchtet wird, ausgeführt sein.

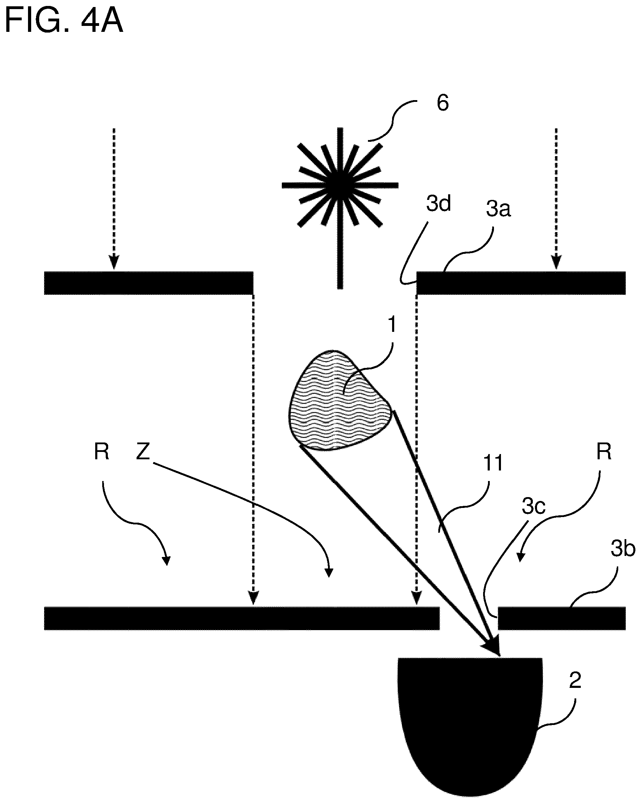

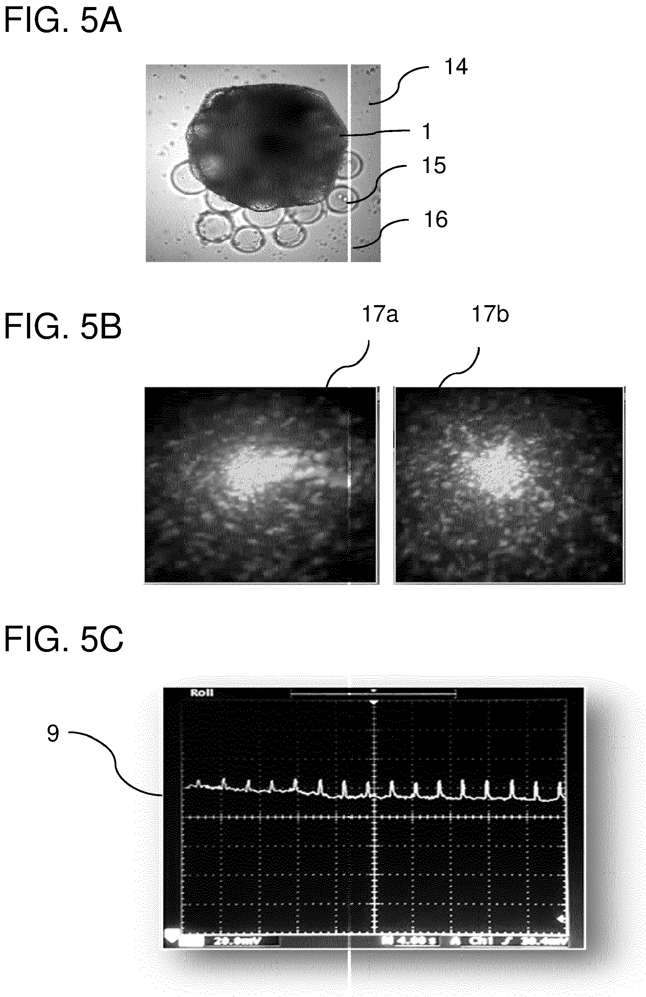

Die Lichtquelle 6 kann, muss aber keine kohärente Lichtquelle, z. B. ein Laser, sein. Lediglich die Ausführungsvarianten, die ein Beugungsmuster der Probe zur Bewegungsdetektion (vgl. Figuren 4A bis 4C, 5A bis 5C) verwenden, benötigen kohärente Strahlung.

Die Darstellung der Optik 7, 8 in Figur 1 ist lediglich schematisch und soll die funktionale Eigenschaft der Optik illustrieren und keine bestimmten optischen Elemente darstellen, da prinzipiell eine Vielzahl konkreter optischer Ausführungsvarianten möglich ist, von denen einige beispielhaft in den nachfolgenden Figuren beschrieben sind.

Diese funktionale Eigenschaft der Optik kann dabei unter Verwendung eines oder mehrerer zweckmäßig angeordneter und ausgestalteter bekannter optischer Bauelemente und Komponenten, wie z. B. Filter, Linsen, Blenden, refraktiver Elemente etc., realisiert werden.

Die Optik 7, 8 umfasst eine auf der Beleuchtungsseite angeordnete Beleuchtungsoptik 7, mittels der die Strahlung 10 der Lichtstrahlenquelle 6 auf die ganze Probe 1 geleitet wird, um die Probe vollständig und möglichst gleichmäßig zu beleuchten. Die Beleuchtungsoptik kann hierfür geeignete optische Elemente bzw. Komponenten zur Strahlformung, wie Blenden, Linsen und/oder Filter, umfassen. Vorteilhaft ist insbesondere die Verwendung eines Axicons.

Die Optik 7, 8 umfasst ferner eine Detektionsoptik 8, mittels der das von der Probe ausgesandte Licht 11, d. h. Licht der Lichtstrahlenquelle 6, das durch eine Wechselwirkung mit der Probe 1 in seiner Strahlrichtung, seinem Polarisationszustand und/oder Beugungsmuster verändert wurde, auf eine Detektionsfläche 2a des Detektors 2 geleitet wird. In Figur 1 ist beispielsweise ein Streuvorgang im Punkt P1 dargestellt, bei dem Licht 11 in Richtung des Detektors 2 gestreut wird und mittels der Detektionsoptik 8 auf den Detektor 2 abgebildet wird. Da die ganze Probe 1 gleichmäßig beleuchtet wird, kann die Wechselwirkung des einfallenden Lichts 10 an jedem beliebigen Punkt innerhalb der Probe 1 stattfinden, so dass die Wechselwirkungen des Lichts mit der Probe auf seiner gesamten Strecke durch die Probe kumuliert vom Detektor 2 gemessen werden.

Die Detektionsoptik 8 kann ferner optische Elemente enthalten, die sicherstellen, dass Licht, das nicht durch eine Wechselwirkung mit der Probe in seiner Strahlrichtung, seinem Polarisationszustand und/oder Beugungsmuster verändert wurde, nicht auf den Detektor 2 trifft. Beispielsweise kann die Detektionsoptik 8 einen Bandpassfilter aufweisen, der vor dem Detektoreingang angeordnet ist und das Raumlicht herausfiltert oder unterdrückt, jedoch Licht mit einer Wellenlänge der Lichtstrahlenquelle 6 durchlässt. Dies setzt voraus, dass eine monochromatische Lichtstrahlenquelle verwendet wird oder ein entsprechender Filter am Ausgang der Lichtstrahlenquelle angeordnet ist, um die Probe nur mit einer bestimmten Lichtwellenlänge zu beleuchten.

Ferner kann die Detektionsoptik 8 mittels Blenden, Linsen etc. Strahlengänge blockieren, bei denen durch die Probe 1 transmittierte Strahlung der Lichtstrahlenquelle 6 auf den Detektor treffen würde und/oder bei denen Licht der Lichtstrahlenquelle 6 unter Umgehung der Probe auf den Detektor 2 treffen würde.

Zusätzlich oder alternativ kann der Detektor auch so angeordnet sein, dass kein Durchlicht 12 auf seine Detektorfläche 2a trifft, z. B. durch seitliche Anordnung des Detektors 2 relativ zum Beleuchtungsstrahlengang 10, wie in Figur 1 illustriert.

Der Detektor 2 ist ausgebildet, in Abhängigkeit von der detektierten Strahlung 11 ein Messsignal 9 zu erzeugen, dessen zeitlicher Verlauf einen zeitlichen Verlauf einer Intensität der detektierten Strahlung 11 angibt.

Das Detektorsignal 9 entspricht somit einer Volumenmessung (engl. full-volume measurement) der Probe. Der Detektor 2 ist vorzugsweise einkanalig, so dass nur eine Messgröße 9 erzeugt wird, die der Schwankung der vom Detektor pro Zeiteinheit erfassten Lichtintensität entspricht. Der Detektor 2 ist z. B. eine herkömmliche Photodiode 2.

Mit der in Figur 1 illustrierten Vorrichtung 100 wird die Probe 1 beleuchtet und das entsprechende Messsignal 9 ausgewertet.

Findet nun eine Bewegung in der Probe 1 statt, z. B. eine Kontraktion im Falle von gezüchtetem Muskelgewebe, dann ändert sich durch die Bewegung in der Probe auch die Wechselwirkung der Probe 1 mit dem auf die Probe treffenden Licht, d. h., der Anteil des auf die Probe treffenden Lichts 11, das durch eine Wechselwirkung mit der Probe 1 in seiner Strahlrichtung, seinem Polarisationszustand und/oder Beugungsmuster verändert wird, ändert sich und erzeugt eine Änderung im Detektorsignal 9. Gemäß dem Verfahren kann somit eine Bewegung in der biologischen Probe anhand der Schwankung des Detektorsignals 9 detektiert werden.

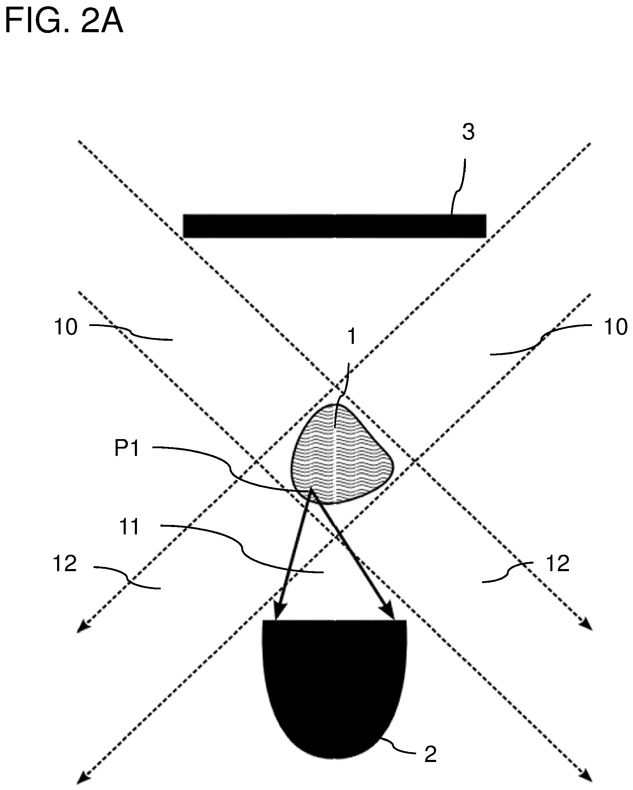

Nachfolgend werden einige beispielhafte Ausführungsformen beschrieben, die konkrete Ausgestaltungen des in Figur 1 dargestellten Lösungsansatzes darstellen. In den Figuren 2A bis 3C ist die Lichtstrahlenquelle 6 nicht dargestellt, befindet sich aber oberhalb der gezeigten Optik- und Detektoranordnung, was aus dem Strahlengang 10 ersichtlich ist.

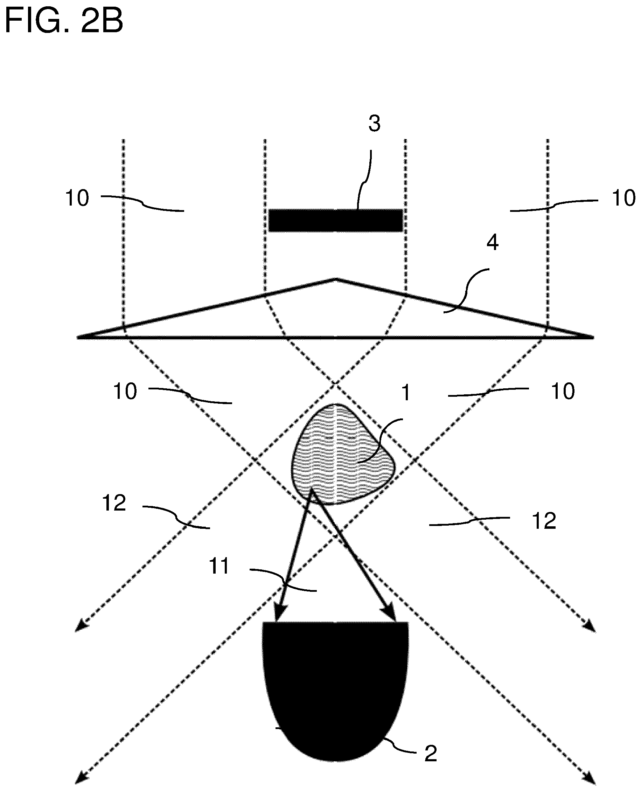

Die Figuren 2A bis 2E zeigen nicht erfindungsgemäßen Ausführungsformen, die Streulicht der Probe zur Bewegungsdetektion verwenden. In den Figuren 2A und 2B ist der Detektor 2 zur Durchlichtdetektion von Streulicht 11 der Probe 2 in Durchlichtrichtung zur Probe 1 angeordnet. Um zu verhindern, dass durch die Probe 2 transmittierte Strahlen auf den Detektor 2 treffen, ist in Figur 2A eine Blende 3 angeordnet, die Lichtstrahlen blockiert, die als durch die Probe 2 transmittierte Strahlen auf den Detektor 2 treffen würden oder die seitlich an der Probe vorbei auf den Detektor 2 treffen könnten. Die Beleuchtungsoptik in Form der Blende 3 lässt also nur solche durch die Probe transmittierte Strahlung 12 zu, die nicht auf den Detektor 12 trifft.

Die Besonderheit der in Figur 2B dargestellten Ausführungsvariante ist, dass statt einer großen Blende 3 eine kleinere Blende 3 verwendet wird, der ein refraktives optisches Element, z. B. eine Linse, ein Axicon etc. nachgeordnet ist, das den durch die Blende 3 durchgelassen Beleuchtungsstrahlengang 10 so in seiner Richtung verändert, dass durch die Probe 2 transmittierte Strahlen 12 nicht auf den Detektor 2 treffen können.

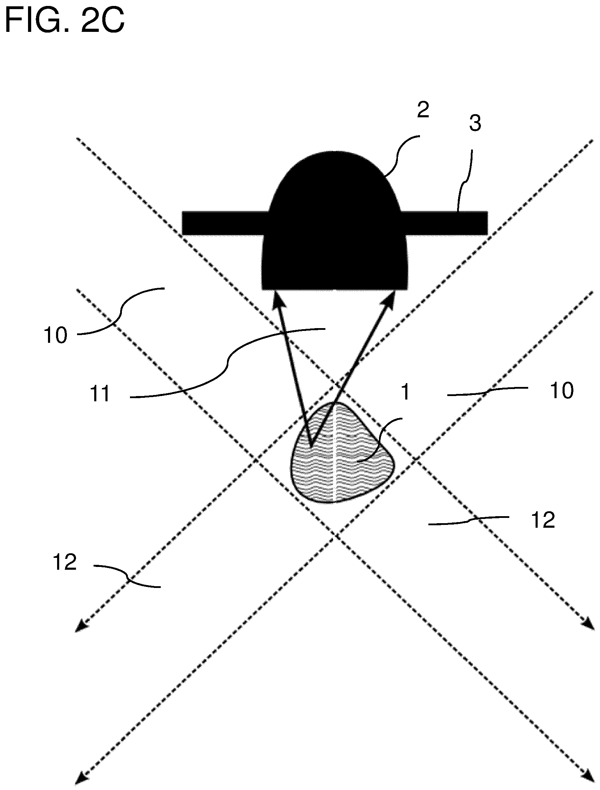

Die Besonderheit der in Figur 2C dargestellten Ausführungsvariante ist, dass der Detektor 2 zur Epidetektion von Streulicht in Form von Rückstrahlung der Probe 1 auf der gleichen Seite der Probe 1 wie die Lichtstrahlenquelle 6 angeordnet ist, wobei die Detektorfläche 2a wiederum der Probe 2 zugewandt ist. Dies bietet den Vorteil, dass keine Blende zum Blockieren von Durchlicht notwendig ist. Vorliegend ist dennoch eine den Detektor 2 umgebende Blende vorgesehen, die Einflüsse von störenden Lichteinflüssen reduzieren kann.

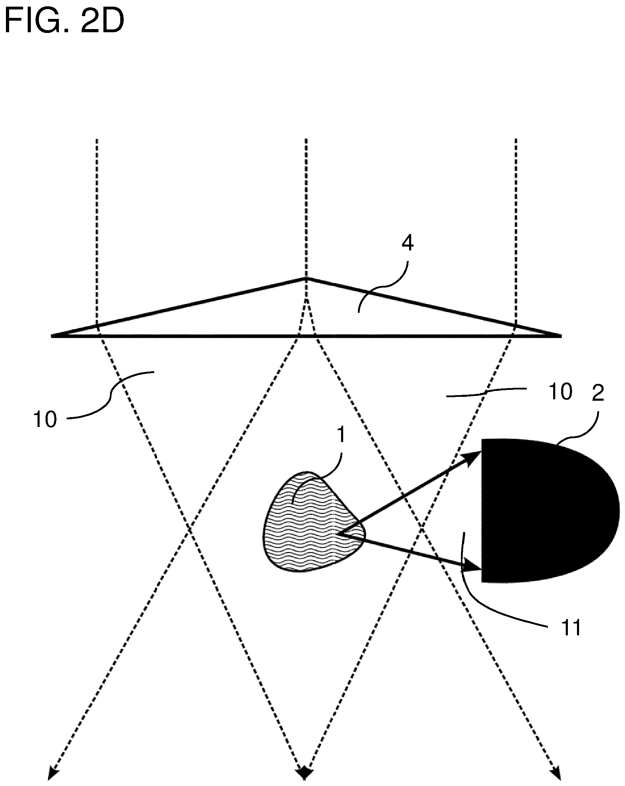

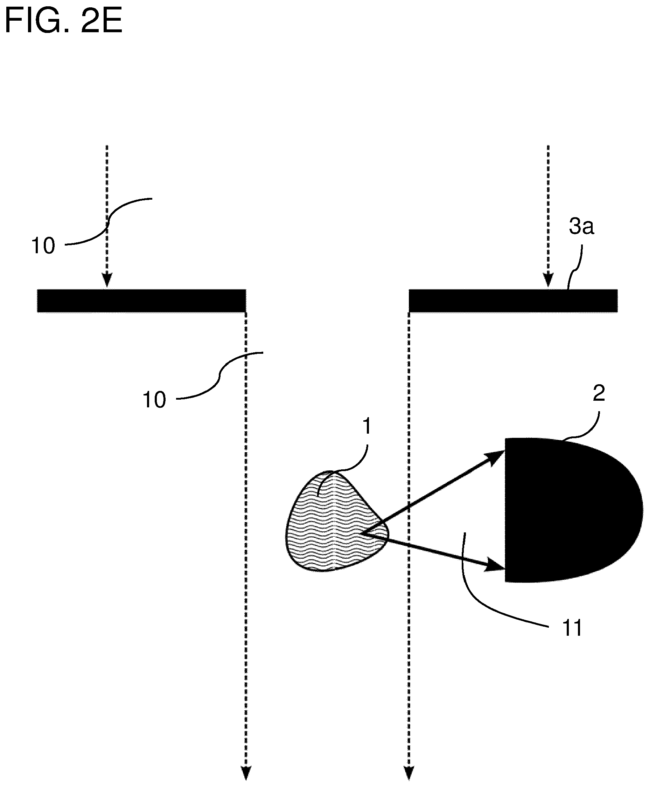

Die Besonderheit der in den Figuren 2D und 2E dargestellten Ausführungsvarianten ist, dass seitliches Streulicht 11 detektiert wird. Hierzu ist der Detektor 2 seitlich zur Beleuchtungsrichtung angeordnet. In Figur 2D ist zwischen Lichtstrahlenquelle und Probe 1 ein refraktives optisches Element, z. B. eine Linse oder Prisma 4, angeordnet, das die Richtung des Strahlengangs so verändert, dass Licht des Beleuchtungsstrahlengangs 10 nicht direkt, d. h. unter Umgehung der Probe 1, auf den Detektor 2 treffen kann.

Die in Figur 2E dargestellte Ausführungsvariante unterscheidet sich von der Variante in Figur 2E dadurch, dass statt des refraktiven optischen Elements 4 eine Blende 3a verwendet wird, die eine Öffnung zur Verengung des Strahlengangs 10 aufweist, so dass wiederum Licht des Beleuchtungsstrahlengangs 10 nicht direkt, d. h. unter Umgehung der Probe 1, auf den Detektor 2 treffen kann.

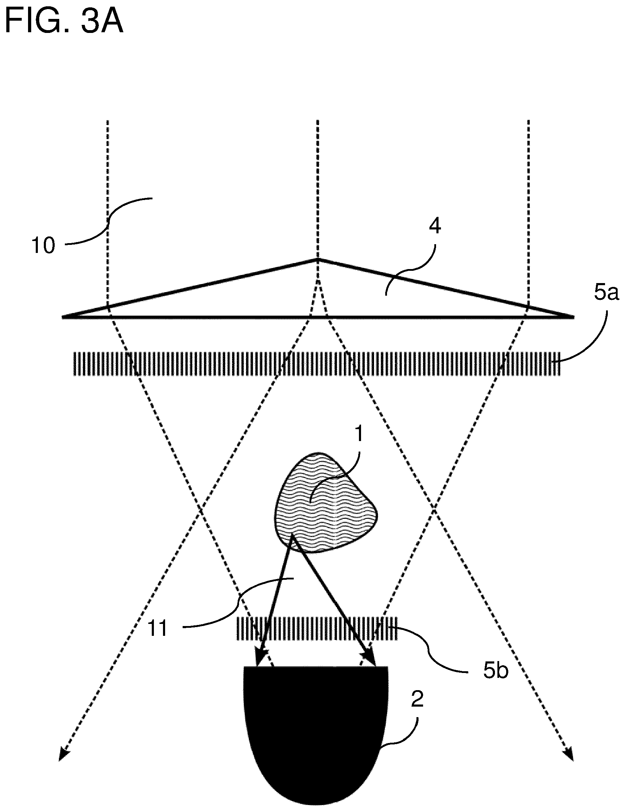

Die Figuren 3A bis 3C zeigen nicht erfindungsgemäßen Ausführungsformen, die polarisiertes Licht zur Bewegungsdetektion verwenden. Die Beleuchtungsoptik umfasst hierzu einen ersten Polarisationsfilter 5a, der zwischen der Lichtstrahlenquelle und der Probe 1 angeordnet ist. Die Detektionsoptik umfasst einen zweiten Polarisationsfilter 5b, der eine unterschiedliche Polarisationsrichtung im Vergleich zum ersten Polarisationsfilter 5a aufweist und zwischen Probe 1 und Detektor 2, vorzugsweise am Detektoreingang, angeordnet ist.

Der Detektor 2 und der zweite Polarisationsfilter 5b können in Bezug auf die Lichtstrahlenquelle auf der gegenüberliegenden Seite der Probe 1, seitlich von der Probe 1 oder auf der gleichen Seite wie die Lichtstrahlenquelle angeordnet sein, was durch die unterschiedlichen Varianten in den Figuren 3A bis 3C dargestellt ist.

Aufgrund der unterschiedlichen Polarisationsrichtung der beiden Polarisationsfilter 5a und 5b lässt der Polarisationsfilter 5b nur Licht durch, das durch eine Wechselwirkung mit der Probe "depolarisiert" wurde. Die Anordnung der beiden Polarisationsfilter 5a und 5b stellt somit sicher, dass kein durch die Probe transmittiertes Licht oder Licht, dass die Probe umgangen hat, detektiert wird.

Durch eine Bewegung in der Probe ändert sich der Anteil an depolarisiertem Licht und führt zu einer Schwankung im Detektorsignal, so dass aus der Schwankung des Detektorsignals wiederum direkt eine Bewegung in der Probe 1 erkannt werden kann.

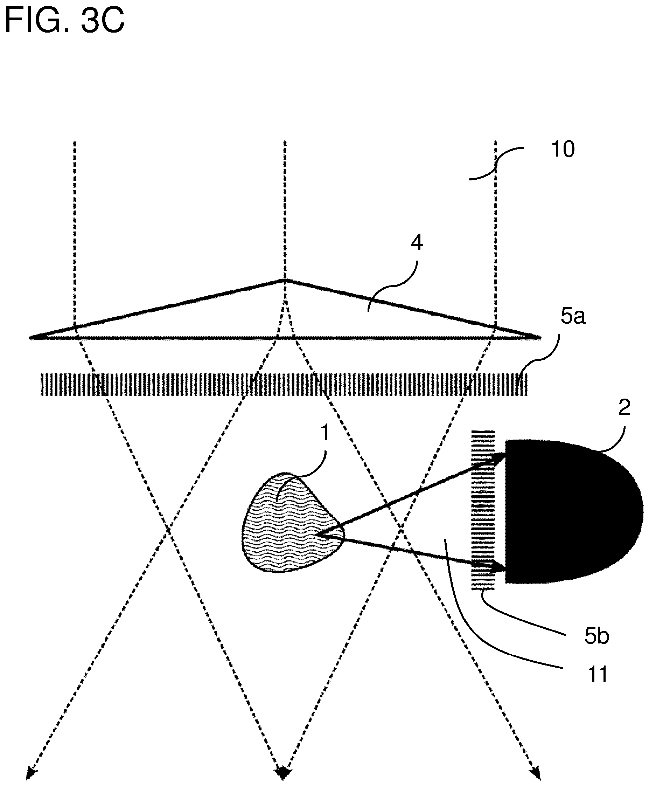

Zur Reduzierung von Streulichteffekten kann vor dem ersten Polarisationsfilter 5a ein refraktives optisches Element 4, z. B. eine konvexe Linse oder ein Prisma, angeordnet sein, das den Beleuchtungsstrahlengang 10 auf die Probe fokussiert (Figuren 3A und 3C).

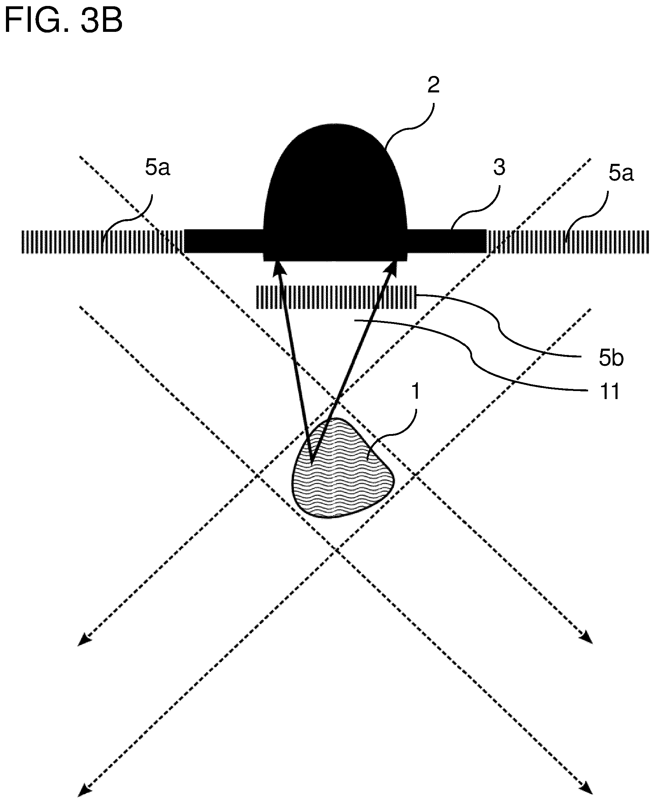

In der Ausführungsvariante der Figur 3B ist der Detektor 2 zur Epidetektion von Streulicht in Form von Rückstrahlung der Probe 1 auf der gleichen Seite der Probe 1 wie die Lichtstrahlenquelle 6 angeordnet. Hier kann eine den Detektor 2 umgebende Blende 3 vorgesehen sein, die Einflüsse von störenden Lichteinflüssen reduzieren kann.

Die Figuren 4A bis 4C zeigen Ausführungsformen zur Verständnis der Erfindung, die ein Beugungsmuster der Probe, z. B. in Form eines Speckle-Musters, zur Bewegungsdetektion verwenden. Die Lichtquelle 6 ist eine kohärente Lichtquelle, z. B. eine Laserdiode. Das an der Probe gebeugte Licht der Lichtstrahlenquelle 6 erzeugt ein Beugungsmuster mit einem Zentrum Z hoher Intensität und einem Randbereich R niedriger Intensität.

Die Detektionsoptik umfasst eine vor dem Detektor 2 angeordnete Lochblende 3b, die so zwischen der Probe 1 und dem Detektor 2 angeordnet ist, dass ein Loch 3c der Lochblende 3b in dem Randbereich R des von der Probe erzeugten Beugungsmusters angeordnet ist.

Gemäß der Variante der Figur 4A umfasst die Beleuchtungsoptik eine zwischen der Lichtstrahlenquelle und der Probe angeordnete Blende 3a, die so ausgeführt ist, dass eine durch eine Blendenöffnung 3d der Blende 3a austretende Strahlung der Lichtstrahlenquelle 6 nicht direkt auf das Loch 3c der Lochblende 3b trifft.

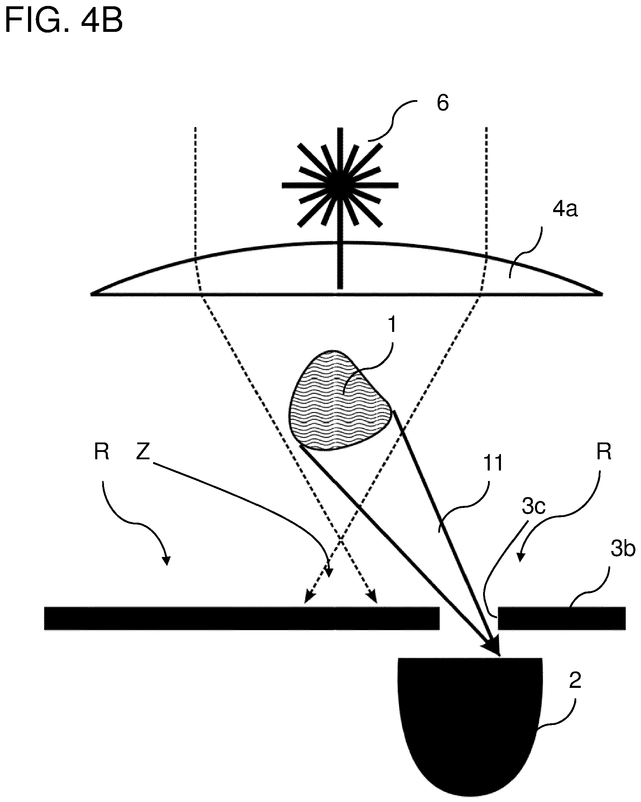

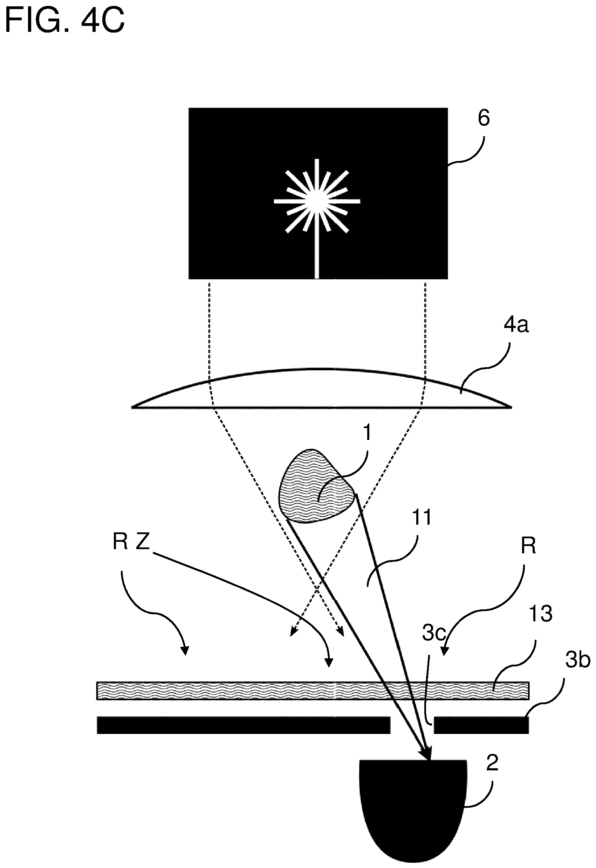

Gemäß den Varianten der Figuren 4B und 4C umfasst die Beleuchtungsoptik ein zwischen der Lichtstrahlenquelle 6 und der Probe 1 angeordnetes refraktives optisches Element 4a, z. B. eine konvexe Linse, das so ausgeführt ist, dass durch das refraktive optische Element 4a gebeugte Strahlung nicht direkt, d.h. unter Umgehung der Probe, auf das Loch 3c der Lochblende 3b trifft.

In der Variante der Figur 4C ist ferner ein Bandpassfilter 13 zwischen Probe 1 und Lochblende 3b angeordnet, der Raumlicht, das nicht der Wellenlänge der Lichtstrahlenquelle 6 entspricht, herausfiltert. Mit dem in Figur 4C gezeigten Aufbau lassen sich derzeit Herzmuskelschläge mit einer der visuellen Beobachtung (am Mikroskop) vergleichbaren Empfindlichkeit nachweisen.

Figur 5A zeigt beispielhaft eine Probe 1 in Form eines Zellhaufens. Der Zellhaufen befindet sich in einem hängenden Tropfen, von dem in Figur 5A nur ein Teil zu sehen ist, der in einer Kavität einer Hängender-Tropfen-Multititer-Platte ausgebildet ist. Die Probe ist besteht aus einem aus Stammzellen ausdifferenzierten Herzmuskelgewebemodell, das auf Trägerbeads 15 in Form von Alginat anhaftet. Die in Figur 5A gezeigte Probe hat einen Durchmesser von ca. 1 Millimeter.

Die Probe 1 wird mit einer Laserdiode mit Licht der Wellenlänge 650nm vollständig beleuchtet. Die auf unterschiedlichen Größenskalen strukturierte Probe 1 beugt das kohärente Licht auf vielfältige Weise und erzeugt in Durchlichtrichtung ein komplexes Beugungsmuster (sog. "Speckle-Muster"). Die durch die lokalen Herzmuskelkontraktionen hervorgerufenen geringfügigen Verformungen dieser Strukturen im Gewebe führen zu einer Veränderung des gesamten Speckle-Musters.

Figur 5B zeigt beispielhaft die Bilder 17a und 17b, die zwei unterschiedliche Zustände eines Speckle-Musters bei minimaler und maximaler Auslenkung der Kontraktion zeigen. Die Bilder 17a und 17b dienen lediglich zur Verdeutlichung und stammen von einem anderen Experiment und zeigen nicht das Speckle-Muster der Probe 1 aus Figur 5A. Das Messprinzip ist jedoch das gleiche. Über einen Raumfilter, z. B. die Lochblende 3b, wird eine Stelle im Randbereich R des Beugungsmusters 17a, 17b auf den Detektor 2 abgebildet. Die Veränderung des Musters 17a, 17b führt nun zu einer schwankenden Lichtmenge, die von der Lochblende 3b durchgelassen wird, und somit zu einem schwankenden Detektorsignal 9, was in Figur 5C dargestellt ist. Die periodische Schwankung des Signals 9 entspricht den periodischen Kontraktionen im Muskelgewebe. Die Darstellung in Figur 5C dient lediglich der Verdeutlichung, zeigt aber wiederum kein Messsignal, das bei der Beleuchtung der in Figur 1A gezeigten Probe gemessen wurde.

Obwohl die Erfindung unter Bezugnahme auf bestimmte Ausführungsbeispiele beschrieben worden ist, ist es für einen Fachmann ersichtlich, dass verschiedene Änderungen ausgeführt werden können und Äquivalente als Ersatz verwendet werden können, ohne den Bereich der Erfindung zu verlassen. Zusätzlich können viele Modifikationen ausgeführt werden, ohne den zugehörigen Bereich zu verlassen. Folglich soll die Erfindung nicht auf die offenbarten Ausführungsbeispiele begrenzt sein, sondern soll alle Ausführungsbeispiele umfassen, die in den Bereich der beigefügten Patentansprüche fallen.

## Patentansprüche

1. Vorrichtung zur kontaktfreien In-Vitro-Detektion einer Bewegung in einer biologischen Probe mit räumlicher Ausdehnung in Form einer dreidimensionalen Zell- und/oder Gewebekultur oder eines Zellhaufens oder einer Probe aus freischwimmenden Mikroorganismen, wobei die Vorrichtung zur parallelen optische Detektion einer Bewegung in mehreren voneinander getrennten biologischen Proben ausgebildet ist, umfassend:
- eine Aufnahme (15, 16) für die biologische Probe (1), wobei die Aufnahme für die Proben eine Multiwell-Platte oder eine Hängender-Tropfen-Multiwell-Platte ist, die eine Mehrzahl von in Reihen und Spalten angeordnete Kavitäten zur Aufnahme der Proben aufweist,
- eine Lichtstrahlenquelle (6), die zur Beleuchtung der Proben in den Kavitäten als ein Laserdiodenarray ausgeführt ist, wobei ein Rasterabstand der einzelnen Laserdioden dem Rasterabstand der Kavitäten der Multiwell-Platte entspricht und Linsen das Licht der Laserdioden in die Kavitäten leiten,
- einen Detektor (2), wobei der Detektor als Detektorarray, vorzugsweise als Photodiodenarray, ausgeführt ist, wobei ein Rasterabstand der einzelnen Detektoren einem Rasterabstand der Kavitäten der Multiwell-Platte entspricht,
- eine Optik (7, 8), die ausgebildet ist, die ganze Probe in der Aufnahme mit von der Lichtstrahlenquelle ausgehender Strahlung zu beleuchten und zumindest einen Teil der Strahlung (11) der Lichtstrahlenquelle (6), die an einer beliebigen Stelle innerhalb der Probe (1) durch eine Wechselwirkung mit der Probe (1) in ihrem Beugungsmuster verändert wurde, auf eine Detektionsfläche (2a) des Detektors (2) zu leiten,
wobei der Detektor (2) ausgebildet ist, in Abhängigkeit von der detektierten Strahlung (11) ein Messsignal (9) zu erzeugen, dessen zeitlicher Verlauf einen zeitlichen Verlauf der Intensität der detektierten Strahlung angibt und/oder aus dem der zeitliche Verlauf der Intensität der detektierten Strahlung ableitbar ist, wobei die Vorrichtung ferner eine Auswerteeinheit umfasst, die eingerichtet ist, eine Bewegung in der biologischen Probe durch Anzeige und/oder Auswertung einer zeitlichen Veränderung der detektierten Strahlung zu detektieren.

2. Vorrichtung nach Anspruch 1, **dadurch gekennzeichnet, dass** die Optik (7, 8) ausgebildet ist und/oder der Detektor (2) relativ zum Beleuchtungsstrahlengang (10) und der Probe (1) so angeordnet ist, dass keine Strahlengänge existieren, bei denen durch die Probe (1) transmittierte Strahlung (12) der Lichtstrahlenquelle (6) auf den Detektor (2) trifft und/oder bei denen Licht der Lichtstrahlenquelle unter Umgehung der Probe auf den Detektor trifft.

3. Vorrichtung nach einem der vorhergehenden Ansprüche, **dadurch gekennzeichnet, dass** die Optik einen vor dem Detektor (2) angeordneten Bandpassfilter (13) aufweist, der zur Raumlichtunterdrückung ausgebildet ist.

4. Vorrichtung nach einem der vorhergehenden Ansprüche, **dadurch gekennzeichnet, dass** die Aufnahme der Probe eine Hängender-Tropfen-Multiwell-Platte ist

5. Vorrichtung nach einem der vorhergehenden Ansprüche, **dadurch gekennzeichnet,**
a) **dass** die Halterung des Laserdiodenarrays aus einem wärmeleitfähigen Material, vorzugsweise aus Aluminium, ausgeführt ist; und/oder
b) **dass** die Optik ein Mikrolinsenarray umfasst, wobei jede Linse des Linsenarrays einer der Laserdioden zugeordnet ist.

## Claims

1. A device for non-contact in vitro detection of a movement in a biological sample with spatial expansion in the configuration of a three-dimensional cell and/or tissue culture or a cell cluster or a sample of free-floating microorganisms, wherein the device is configured for parallel optical detection of a movement in a plurality of biological samples separated from each other, comprising:
- a receptacle (15, 16) for the biological sample (1), wherein the receptacle for the samples is a multi-well plate or a hanging-drop multi-well plate comprising a plurality of cavities arranged in rows and columns for receiving the samples,
- a light beam source (6) which is configured as a laser diode array for illuminating the samples in the cavities, wherein a grid spacing of the individual laser diodes corresponds to the grid spacing of the cavities of the multiwell plate and lenses guide the light of the laser diodes into the cavities,
- a detector (2), wherein the detector is configured as a detector array, preferably as a photodiode array, wherein a grid spacing of the individual detectors corresponds to a grid spacing of the cavities of the multiwell plate,
- optics (7, 8) which is configured to illuminate the entire sample in the image with radiation emanating from the light beam source and to direct at least a portion of the radiation (11) from the light beam source (6), the diffraction pattern of which has been altered at any point within the sample (1) by an interaction with the sample (1), onto a detection surface (2a) of the detector (2),
wherein the detector (2) is configured to generate a measurement signal (9) dependent on the detected radiation (11), the time characteristic of which indicates a time characteristic of the intensity of the detected radiation and/or from which the time characteristic of the intensity of the detected radiation can be derived, wherein the device further comprises an evaluation unit which is configured to detect a movement in the biological sample by displaying and/or evaluating a temporal change in the detected radiation.

2. Device according to claim 1, **characterised in that** the optics (7, 8) are configured and/or the detector (2) is arranged relative to the illumination beam path (10) and the sample (1) in such a way that no beam paths exist in which radiation (12) from the light beam source (6) transmitted through the sample (1) strikes the detector (2) and/or in which light from the light beam source strikes the detector while bypassing the sample.

3. Device according to one of the preceding claims, **characterised in that** the optics comprise a bandpass filter (13) arranged in front of the detector (2), which is configured for spatial light suppression.

4. Device according to one of the preceding claims, **characterised in**
**that** the receptacle for the sample is a hanging-drop multi-well plate.

5. A device according to any one of the preceding claims, **characterised in that,**
a) that the holder of the laser diode array is provided from a thermally conductive material, preferably aluminium; and/or
b) that the optics comprises a microlens array, wherein each lens of the lens array is associated with one of the laser diodes.

## Revendications

1. Dispositif de détection in vitro sans contact d'un mouvement dans un échantillon biologique à expansion spatiale sous la forme d'une culture cellulaire et/ou tissulaire tridimensionnelle, ou d'un amas cellulaire, ou d'un échantillon de microorganismes nageant librement, le dispositif étant conçu pour la détection optique parallèle d'un mouvement dans plusieurs échantillons biologiques séparés les uns des autres, comprenant :
- un logement (15, 16) destiné à l'échantillon biologique (1), le logement à échantillons étant une plaque multi-puits ou une plaque multi-puits à gouttes suspendues qui comporte une pluralité de cavités agencées en rangées et en colonnes pour la réception des échantillons,
- une source de rayonnement lumineux (6) qui est réalisée sous la forme d'un réseau de diodes laser pour éclairer les échantillons contenus dans les cavités, un espacement de trame des différentes diodes laser correspondant à l'espacement de trame des cavités de la plaque multi-puits, et des lentilles guidant la lumière des diodes laser vers les cavités,
- un détecteur (2), le détecteur étant réalisé sous la forme d'un réseau de détecteurs, de préférence sous la forme d'un réseau de photodiodes, un espacement de trame des détecteurs individuels correspondant à un espacement de trame des cavités de la plaque multi-puits,
- une optique (7, 8) qui est conçue pour éclairer la totalité de l'échantillon contenu dans le logement avec un rayonnement provenant de la source de rayonnement lumineux et pour guider au moins une partie du rayonnement (11) provenant de la source de rayonnement lumineux (6), dont le diagramme de diffraction a été modifié par une interaction avec l'échantillon (1) en un point quelconque à l'intérieur de l'échantillon (1), vers une surface de détection (2a) du détecteur (2),
le détecteur (2) étant conçu pour générer, en fonction du rayonnement détecté (11), un signal de mesure (9) dont l'évolution temporelle indique l'évolution temporelle de l'intensité du rayonnement détecté et/ou dont peut être déduite l'évolution temporelle de l'intensité du rayonnement détecté, le dispositif comprenant en outre une unité d'évaluation qui est conçue pour détecter un mouvement dans l'échantillon biologique par affichage et/ou évaluation d'une variation temporelle du rayonnement détecté.

2. Dispositif selon la revendication 1, **caractérisé en ce que** l'optique (7, 8) est conçue et/ou le détecteur (2) est agencé par rapport au chemin du faisceau d'éclairage (10) et à l'échantillon (1) de telle sorte qu'il n'existe aucun chemin de faisceau par l'intermédiaire duquel un rayonnement (12) provenant de la source de rayonnement lumineux (6) et transmis à travers l'échantillon (1) atteint le détecteur (2) et/ou par l'intermédiaire duquel la lumière provenant de la source de rayonnement lumineux atteint le détecteur en contournant l'échantillon.

3. Dispositif selon l'une quelconque des revendications précédentes, **caractérisé en ce que** l'optique comporte un filtre passe-bande (13) agencé devant le détecteur (2), lequel est conçu pour supprimer la lumière ambiante.

4. Dispositif selon l'une quelconque des revendications précédentes, **caractérisé en ce que** le logement à échantillons est une plaque multi-puits à gouttes suspendues.

5. Dispositif selon l'une quelconque des revendications précédentes, **caractérisé**
a) **en ce que** le support du réseau de diodes laser est réalisé en un matériau thermoconducteur, de préférence en aluminium ; et/ou
b) **en ce que** l'optique comprend un réseau de microlentilles, chaque lentille du réseau de lentilles étant associée à l'une des diodes laser.
